# EUROPÄISCHE PATENTANMELDUNG

(11) **EP 1 955 713 A1**
(43) Veröffentlichungstag der Anmeldung: **13.08.2008**
(21) Anmeldenummer: 08002115.7
(22) Anmeldetag: 18.07.2003
(51) Int. Cl.: A61K 47/48

(54) **Bisacylpropylcystein-Konjugate und deren Verwendung**

(30) Priorität: 19.07.2002 EP 02016066
(62) Teilanmeldung aus: 03765055.3
(71) Anmelder: Helmholtz-Zentrum für Infektionsforschung GmbH, 38124 Braunschweig (DE)
(72) Erfinder: Mühlradt, Peter, 38114 Braunschweig (DE); Morr, Michael, 38302 Wolfenbüttel (DE)
(74) Vertreter: Kröncke, Rolf

(57) **Zusammenfassung**

Es werden neue Lipopeptid-Konjugate vorgeschlagen, bei denen ein doppelt fettsäuresubstituiertes Cystein über die Carboxylgruppe an einen gut wasserlöslichen und physiologisch verträglichen sowie nicht immunogenen polymeren Konjugatrest gebunden ist. Die neuen Konjugate zeigen sehr gute makrophagenstimulierende Wirkung und benötigen keine zusätzliche Lösungsvermittlung. Sie bieten sich für zahlreiche Verwendungen an, insbesondere zur Stimulierung von Makrophagen, zur Stimulierung der Antikörper-Synthese, zur Infektionsabwehr, zur Immunstimulanz, insbesondere gegenüber Tumoren, zur Vorbeugung und Behandlung von septischem Schock, zur Wundheilung und als Adjuvans für Impfstoffe.

## Beschreibung

Die Erfindung betrifft neue Bisacyloxypropylcystein-Konjugate und deren Verwendung, auch in Form pharmazeutischer Zusammensetzungen.

Es ist seit langem bekannt, dass bestimmte Lipopeptide Makrophagenaktivatoren sind (Hoffmann, P., S. Heinle, U.F. Schade, H. Loppnow, A.J. Ulmer, H.-D. Flad, G. Jung, and W. Bessler, 1988, "Stimulation of human and murine adherent cells by bacterial lipoprotein and synthetic Bisacyloxypropylcysteine analogues", Immunobiol. 177:158-170). Innerhalb dieser Klasse von Makrophagenaktivatoren sind insbesondere auch mehrfach an einem Propylcysteinrest fettsäuresubstituierte (acyloxy-substituierte) Peptide oder Proteine mit physiologischer Wirkung bekannt.

Peptide und darunter besonders Lipopeptide mit längeren Fettsäureketten haben jedoch für pharmazeutische und verwandte Anwendungen am menschlichen und tierischen Körper häufig den gravierenden Nachteil zu geringer Wasserlöslichkeit, was ihre Wirksamkeit und ihr Einsatzgebiet stark einschränkt.

Diese Probleme können durch Konjugieren der Proteine und Peptide an wasserlösliche Polymere gelöst werden. Aus der EP 0 510 356 B1 sind beispielsweise Polyethylenglykol-Proteinkonjugate bekannt, in denen ein Protein über einen Linker an Polyethylenglykol gebunden ist und hierdurch wesentlich wasserlöslicher gemacht wird. In der EP 0 510 356 B1 sind zahlreiche weitere Schriften genannt, in denen das Konjugieren von Peptiden oder Proteinen an wasserlösliche Polymere, hier besonders an Polyethylenglykol, beschrieben wird. Die Konjugation mit PEG wird heute auch als "Pegylieren" bezeichnet.

Auch sind bereits in oben beschriebener Weise pegylierte Makrophagenaktivatoren bekannt. Kommerziell erhältlich ist beispielsweise ein dreifach an einem Propyl-Cysteinrest acyloxy-substituiertes PEG, Tripalmitoyl-S-Glyceryl-Cystein-Polyethylen-glykol, das als PAM₃-Cys-PEG bezeichnet werden' kann.

PAM₃-Cys-PEG besitzt die Struktur 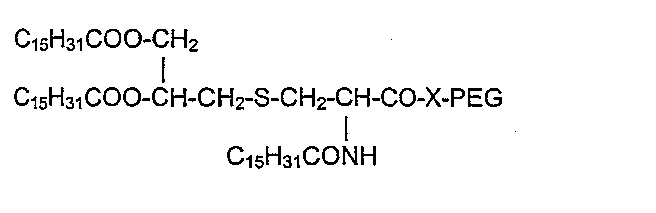 wobei das fettsäuresubstituierte Propylcystein an Position X über einen zweiwertigen Rest wie -NH-, -OCO-, -S-, -O-, oder dergleichen mit dem Polyethylenglykol konjugiert ist.

Trotz der stark verbesserten Wasserlöslichkeit von PAM₃-Cys-PEG gegenüber den entsprechenden PAM₃-Cys-Peptiden ist häufig bei der Verwendung dieser Substanz als Makrophagenaktivator die Zugabe eines organischen Lösungsvermittlers erforderlich oder zumindest sehr empfehlenswert, da die Makrophagenaktivierung sonst geringer wäre. Lösungsvermittler wie Octyl-Glucosid sind jedoch aus pharmakologische Sicht nicht ganz unproblematisch.

Es besteht daher weiterhin ein starkes Bedürfnis für neue, physiologisch gut verträgliche, insbesondere nicht-immunogene, gut wasserlösliche und aktive Makrophagenaktivatoren. Die Aufgabe der Erfindung besteht daher darin, einen solchen neuen gut verträglichen, wasserlöslichen und hochaktiven Makrophagenaktivator zur Verfügung zu stellen.

Die Aufgabe wird gelöst durch ein Bisacyloxypropylcystein-Konjugat gemäß Formel (1), 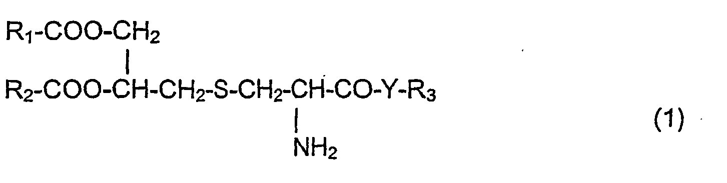 , wobei R₁ und R₂ gleich oder verschieden sein können und über die Carboxylgruppe gebundene Fettsäurereste bedeuten,
Y = -NH-, -O-, -S- oder -OCO- ist,
R₃ ein kovalent, ionisch oder assoziativ gebundener Konjugatrest ist, insbesondere ein wasserlösliches und physiologisch verträgliches, kovalent oder ionisch gebundenes Polymer, insbesondere kovalent gebundenes Polyethylenglykol (Polyoxyethylen),
-(CH₂-CH₂-O)ₘ-CH₂-CH₂-X,
mit X = OR, NR₂, SR, COOR und
R = H, Benzyl-, C₁₋₆-Alkyl, wobei mehrere Reste R gleich oder verschieden sein können,
ein Polyoxyethylen-Polyoxypropylen-Copolymer, ein Dextran, ein Zucker, ein Polyvinylpyrrolidon, ein Alginat, ein Pektin oder ein Kollagen,
und wobei der polymere Rest R₃ ein-, zwei- oder mehrfach mit 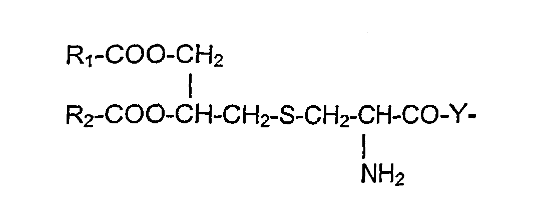 substituiert ist.

Das erfindungsgemäße Konjugat kann sowohl als Racemat als auch als optisch reine Substanzen vorliegen.

Bei dem erfindungsgemäßen Bisacyloxypropylcystein-Konjugat handelt es sich vorzugsweise um ein S-[*2,3*-bis(acyloxy)-(2*S*)-propyl]-L-cysteinyl-carboxy-polyethylenglykol (BAP-Cys-PEG), bei dem das Polyethylenglykol über die Carboxylgruppe des Cysteins angebunden ist und die Aminogruppe des Cysteins frei bleibt. In einer anderen Ausführungsform handelt es sich bei dem erfindungsgemäßen Bisacyloxypropylcystein-Konjugat vorzugsweise um ein S-[*2*,*3*-bis(acyloxy)-(2*R*)-propyl]-L-cysteinyl-carboxy-polyethylenglykol (BAP-Cys-PEG), bei dem das Polyethylenglykol über die Carboxylgruppe des Cysteins angebunden ist und die Aminogruppe des Cysteins frei bleibt. Unter Erhalt der Funktion bzw. hier der physiologischen Wirkung können dem Chemiker bekannte, übliche Modifikationen und Substitutionen an dem Molekül vorgenommen werden.

Die Reste R_{1,2} des erfindungsgemäßen Bisacyloxypropylcystein-Konjugats können gleich oder verschieden sein. Derzeit bevorzugt sind C₇₋₂₅-, vorzugsweise C₈₋₂₂-Alkyl, -Alkenyl oder -Alkinylgruppen, wobei die ungesättigten Positionen vorzugsweise in cis-Konfiguration vorliegen. Die Alkyl-, Alkenyl -und Alkinyl-Reste können verzweigte oder unverzweigte, cyclische oder cycloalkyl-substituierte Reste darstellen. Geeignete Reste R₁, R₂ sind aus der Fettsäurechemie hinlänglich bekannt.

Bezüglich der Gruppe Y kommt es lediglich darauf an, dass eine stabile Verbindung zu dem Konjugatrest R₃ hergestellt wird, damit das Bisacyloxypropylcystein hinlänglich an diesen gebunden und damit wasserlöslich bleibt.

Der Rest R₃ ist vorzugsweise ein Polyethylenglykol-Rest. Allgemein handelt es sich jedoch um einen kovalent, ionisch oder assoziativ gebundenen physiologisch verträglichen Konjugatrest, der geeignet ist, das Bisacyloxypropylcystein in eine aktive wasserlösliche Form zu überführen. Kovalent gebundene Polymere sind derzeit bevorzugt. Alternativ kann der Konjugatrest jedoch auch ein Dextran, ein Zucker, ein Polyvinylpyrrolidon, ein Alginat, ein Pektin oder ein Kollagen sein. Insbesondere Dextran ist als Blutexpander im Einsatz und von seiner physiologischen Verträglichkeit her unbedenklich.

Gerade im Falle hochpolymerer Konjugatreste können auch mehrere BAP-Cys-Einheiten an einen Konjugatrest R₃ gebunden sein.

Das Molekulargewicht des wasserlöslichen Polymerrestes ist vorzugsweise so gewählt ist, dass es pro Bisacyloxypropylcystein-Molekül 100 bis 30 000 Dalton beträgt. Im Falle von Polyethylenglykol ist eine Kettenlänge m von 5 bis 700, vorzugsweise 100 bis 500 bevorzugt, jedoch nicht zwingend.

In einer bevorzugten Ausführungsform ist das S-[*2,3*-bis(acyloxy)-(2*S*)-propyl]-L-cysteinyl-carboxy-polyethylenglykol ein S-[*2,3*-bis(palmitoyloxy)-(2*S*)-propyl]-L-cysteinyl-carboxy-polyethylenglykol. In einer anderen bevorzugten Ausführungsform ist das S-[*2,3-*bis(acyloxy)-(2*R*)-propyl]-L-cysteinyl-carboxy-polyethylenglykol ein S-[*2,3*-bis(palmitoyloxy)-(2*R*)-propyl]-L-cysteinyl-carboxy-polyethylenglykol.

Das Bisacyloxypropylcystein-Konjugat nach dieser Erfindung weist gegenüber vorher bekannten Makrophagenaktivatoren den Vorteil auf, dass es eine gute Wasserlöslichkeit mit einer vergleichsweise sehr hohen Makrophagenaktivität verbindet (siehe unten). Es ist nicht immunogen, so dass bei der Verwendung an Mensch oder Tier keine Antikörperentwicklung gegen das Präparat erfolgt.

Die Erfindung umfasst weiterhin pharmazeutische Zusammensetzungen, die das erfindungsgemäße Bisacyloxypropylcystein-Konjugat enthalten. Derartige Zusammensetzungen schließen unter anderem Lösungen des erfindungsgemäßen Bisacyloxypropylcystein-Konjugats ein. Innerhalb dieser Zusammensetzungen können zusätzliche pharmazeutische Zusatz- und Hilfsstoffe enthalten sein. Insbesondere kann das Bisacyloxypropylcystein-Konjugat an einen pharmazeutisch akzeptablen Träger gebunden oder adsorbiert sein. Geeignete Träger sind dem Fachmann bekannt und stehen auch kommerziell zur Verfügung.

Die pharmazeutische Zusammensetzung liegt vorzugsweise in Form einer für die Injektion, für die Inhalation oder für die intranasale oder topische Applikation geeigneten Formulierung vor, wobei trägergebundene Applikationsformen eingeschlossen sind. Zur lokalen Applikation sind unter anderem Salben, Cremes, Tinkturen, Lösungen und der gleichen vorgesehen, wie dem Fachmann für diesen Zweck bekannt.

Die erfindungsgemäßen Bisacyloxypropylcystein-Konjugate oder die zugehörigen pharmazeutischen Zusammensetzungen können unter anderem zur Stimulierung von Makrophagen, zur Stimulierung der Antikörper-Synthese, zur Infektionsabwehr, zur Immunstimulanz, insbesondere gegenüber Tumoren, zur Vorbeugung und Behandlung von septischem Schock, zur Wundheilung und als Adjuvans für Impfstoffe eingesetzt werden.

Als Adjuvantien werden Substanzen bezeichnet, die bei einer Immunisierung dem eigentlichen Antigen (d.h. der Substanz, die die gewünschte Immunreaktion provoziert) beigefügt werden, um die humorale und/oder zellvermittelte Immunantwort zu verstärken.

Die Verwendung optimaler Adjuvantien spielt bei Stoffen eine entscheidene Rolle. Ohne Adjuvantien verabreichte Antigene vermitteln nur selten eine ausreichende Immunantwort. Darüber hinaus kommt es nicht nur auf die Stärke der hervorgerufenen Immunantwort an, sondern auch auf ihre Qualität. Die Stimulierung eines falschen Immunisierungsmusters kann zu immunpathologischen Reaktionen und Verschlechterung der Infektionssymptome führen. In diesem Zusammenhang kann das Adjuvans helfen, die gewünschte Immunreaktion zu unterstützen.

Die Adjuvantien lassen sich mit den verschiedensten Antigenen zu Impfstoffen kombinieren. Als Antigene können insbesondere Zielantigene für die prophylaktische Behandlung von Infektionskrankheiten, Tumoren, Autoimmunerkrankungen, Allergien sowie chronischer oder aktuter entzündlicher Krankheiten ausgewählt werden. Unter einer Impfung wird auch eine Behandlung mit Antigenen zur Fertilitätskontrolle in menschlichen oder tierischen Populationen verstanden.

Diese Verwendungen umfassen die Aktivierung von Makrophagen/Monozyten oder anderer Zellen, die die Rezeptorkombination Toll-like Rezeptor 2 und 6 tragen, mit allen indirekten Folgen durch Mediatoren in Tier oder Mensch. Dies impliziert die Anwendung als Adjuvans (also als Beimischung zu Vakzinen bzw. Impfstoffen), zur Tumortherapie im weitesten Sinne, einschließlich eines in vitro Primens zu reimplantierender autologer Zellen gegen Tumorantigene oder durch direkte Therapie, die lokal oder systemisch erfolgen kann, zur Erzeugung von Kreuztoleranz gegen Endotoxin und andere entsprechende mikrobielle Komponenten, was einen Schutz vor Sepsis ermöglicht, sowie zur Beschleunigung der Wundheilung.

Fig. 1: Konzentrationsabhängigkeit der Makrophagenaktivierung, gemessen mittels der Stickstoffmonoxid-Produktion (spektroskopisch bestimmt bei OD 550 nm), über der Konzentration an Makrophagenaktivator in Pikomol.

Fig. 2: Beschleunigung der Wundheilung bei diabetischen Mäusen durch dreimalige Applikation von BPP-Cys-PEG. Dreieckige Symbole: mit Carrier behandelte Kontrolltiere; viereckige Symbole: mit BPP-Cys-PEG behandelte Tiere.

Fig. 3: Humorale Antworten stimuliert nach Impfung mit MALP-2 Derivaten und BPP-Cys-PEG als Mukosaladjuvantien mit einer Dosis von 0,05 µg pro Tier pro Immunisierung. Die Mäuse wurden intranasal mit β-Galactosidase (50 µg/Dosis) vermischt mit den obigen Derivaten am Tag 0, 7 und 14 immunisiert. Am Tag 31 nach erster Immunisierung wurden Serumproben entnommen und die Konzentrationen der β-Galactosidase spezifischen Antikörper mit ELISA bestimmt. Die Ergebnisse dargestellt als Endpunkttiter.

Fig. 4: Gesamt β-gal spezifisches IgA in der Lungenspülung von intranasal immunisierten Mäusen. Standardabweichung (SA) ist durch vertikale Linien dargestellt.

Fig. 5: Gesamt β-gal spezifisches IgA in der Vaginalspülung von intranasal immunisierten Mäusen. Standardabweichung ist als vertikale Linien dargestellt.

Fig. 6: β-gal spezifische T-Zell-Proliferationsantworten von Milzzellen von immunisierten Mäuse. Die Zellen wurden in-vitro über 4 Tage mit verschiedenen Konzentrationen von löslichem β-gal restimuliert. Die Ergebnisse sind dargestellt als Verhältnis zwischen den Werten (Durchschnitt von Dreifachbestimmung) von stimulierten und nicht stimulierten Proben (Stimulationsindex).

### BEISPIELE

### 1. Synthese eines erfindungsgemäßen Bisacyloxypropylcysteins mit R₁, R₂ = Palmitoyl, Y = NH und R₃ = PEG

A. Die Synthese von (I) erfolgt nach beschriebener Methode (Metzger, J., Wiesmüller, K.-H., Schaud, R., Bessler, W.G., und Jung, G., 1991, "Synthesis and novel immunologically active tripalmitoyl-S-glycerylcysteinyl Bisacyloxypropylcysteines as useful intermediates for immunogen preparations". Int. J. Pept. Protein Res. 37:46-57; Metzger,J.W., Wiesmüller, K.-H., und Jung, G., 1991, "Synthesis of N-Fmoc protected derivatives of S-(2,3-dihydroxypropyl)-cysteine and their application in peptide synthesis", Int. J. Pept. Protein Res. 38:545-554).

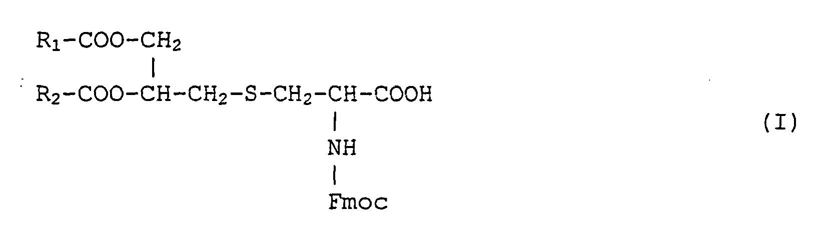

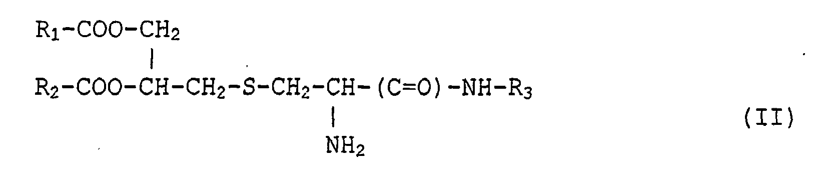

insbesondere mit: R_{1,2} = C₁₅H₃₁- (Palmitoyl-) und R₃ = -(CH₂-CH₂-O)ₓ-CH₂-CH₂-NH₂

B. Die Carboxylgruppe wird anschließend zum Beispiel mit wasserlöslichen NH₂-Gruppen enthaltenden Polymeren, z.B. Diamino-PEG nach bekannten Methoden (Carbodiimid-Synthese) verknüpft.

### Beispiel:

34 mg (38 µmol) (I) werden in 1 ml trockenem Dimethylformamid/Dichlormethan 2:1 gelöst und nacheinander mit 6 µl (38 µmol) Diisopropylcarbodiimid und 6 mg (38 µmol) 1-Hydroxybenzotriazol versetzt. Zu dieser Mischung gibt man 76 mg (38 µmol) Diamino-PEG 2000. Nach 24 Stunden bei Raumtemperatur wird zur Trocknung eingeengt und die Fmoc-Schutzgruppe mit 20 % Piperidin in Dimethylformamid abgespalten. Die Verbindung (II) wird durch Kieselgelchromatographie gereinigt. Charakterisierung durch NMR und Massenspektroskopie. Weitere Reinigung durch HPLC auf C18 Säule bei 40 °C, Puffer 1: 0,1 % TFA in Wasser; Puffer 2: 0,1 % TFA in 2-Propanol. Substanz eluiert bei ca. 80 % V/V Puffer 2.

Gehaltsanalyse erfolgt durch Fettsäurebestimmung mit intern zugemischtem Standard C14, nach Hydrolyse und GLC nach Standardmethoden oder durch Aminogruppenbestimmung mit Fluorescamin.

### C. Biologische Prüfung:

Prinzipiell kann die Makrophagen- und Monozytenaktivierung durch eine Vielzahl von Parametern gemessen werden, wie z.B. durch die Freisetzung von Zytokinen, Chemokinen oder Arachidonsäuremetaboliten in Kulturen von humanen Monozyten oder murinen Peritonealmakrophagen. Der hier verwendete Test beruht auf simultaner Stimulation von Peritonealmakrophagen der Maus mit Interferon-γ und Makrophagenaktivator, z.B. BAP-Cys-PEG zur Freisetzung von Stickstoffmonoxid. (Referenz: Mühlradt, P. F., and Frisch, M. ,1994, "Purification and partial biochemical characterization of a Mycoplasma fermentans-derived substance that activates macrophages to release nitric oxide, tumor necrosis factor, and interleukin-6", Infect. Immun. 62:3801-3807)

### Assay zur Stickstoffmonoxid-Freisetzung:

Kurzgefasst dienten Peritonealmakrophagen von C3H/HeJ-Mäusen als Makrophagenquelle. Diese wurden in 96-well Mikrotiterplatten kultiviert und simultan mit rIFN-γ und einer seriellen Verdünnung an Makrophagenaktivator stimuliert. Soweit erforderlich wurden die Makrophagenaktivatoren im ersten Verdünnungsschritt in 25 mM Octylglucosid gelöst und dann mit Medium weiterverdünnt. Nach einer Inkubationszeit von 45 - 48 Stunden, wurde das Nitrat mit Nitrat-Reduktase reduziert und die Ausgangssubstanz Stickstoffmonoxid mit Griess' Reagenz als Summe aus Nitrat und Nitrit bestimmt. 1 Unit (U)/ml ist definiert als diejenige Verdünnung; bei der eine halbmaximale Stimulation erfolgt.

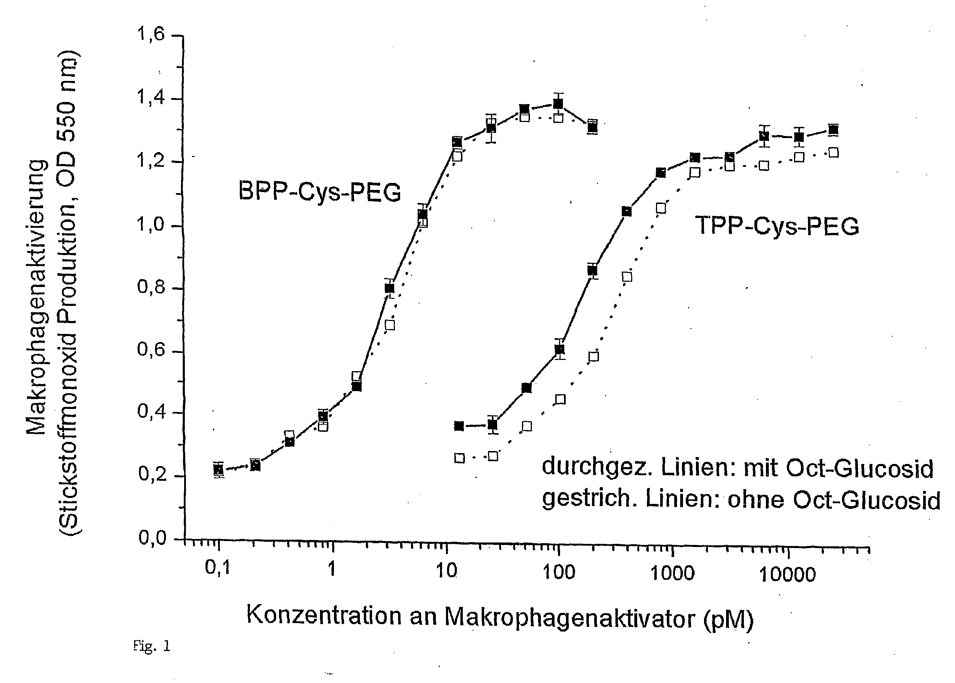

Die Ergebnisse des Makrophagenaktivierungstests sind in Figur 1 dargestellt.

Der Abbildung ist zu entnehmen, dass Bispalmitoyloxypropyl-Cystein-PEG (BPP-Cys-PEG) - ein Makrophagenaktivator gemäß dieser Erfindung - ein deutlich höheres Potential zur Aktivierung von Makrophagen besitzt als das bekannte PAM₃-Cys-PEG (hier als TPP-Cys-PEG bezeichnet ). Die Figur zeigt, dass das gleiche Ausmaß an Makrophagenaktivierung durch BPP-Cys-PEG schon bei einer ca. vierzigfach niedrigeren Konzentration als bei TPP-Cys-PEG erreicht wird.

Die Abbildung zeigt weiter, dass diese ausgezeichnete und unerwartete Aktivierungswirkung bei BPP-Cys-PEG durch Zugabe eines Lösungsvermittlers - hier Octyl-Glucosid - nicht merklich verbessert wird, während zur optimalen Entfaltung der Wirkung von PAM₃-Cys-PEG ein solcher Zusatz erforderlich ist.

Das neue Bisacyloxypropylcystein-Konjugat nach dieser Erfindung bedarf daher keiner zusätzlichen und gegebenenfalls physiologisch nachteiligen Lösungsvermittlung durch ein organisches Lösungsmittel oder Detergenz.

Ein weiterer Vorteil des BAP-Cys-PEG gegenüber dem PAM₃-Cys-PEG ist die größere Zellspezifität, die darauf zurückzuführen ist, dass diese Substanz die Kooperation der Toll-like-Rezeptoren 2 und 6 erfordert, während für eine Stimulation durch PAM₃-Cys-PEG die gleichzeitige Anwesenheit der Toll-like Rezeptoren 1 und 2 auf der zu stimulierenden Zelle ausreicht. Die Expression des Toll-like Rezeptor 6 ist auf bestimmte Zellen beschränkt, während Toll-like Rezeptor 1 ubiquitär von fast allen Körperzellen exprimiert wird.

### 2. Wundheilung bei diabetischen Mäusen

Ähnlich wie bei diabetischen Patienten ist auch bei diabetischen Mäusen die Wundheilung gestört und verläuft langsamer als bei Wildtyp Mäusen. Deshalb ist die diabetische Maus in der Wundheilung ein etabliertes Tiermodell. 20 diabetischen Mäusen (32 C57BKLS/Bom-db) wurde auf dem Rücken eine ca. 4 x 4 cm große Fläche rasiert und diese zwei Tage später mit Veet enthaart und die Creme sorgfältig entfernt. Nach zwei weiteren Tagen wurden die enthaarten Rücken der Mäuse mit Braunol desinfiziert. Nach Einleitung der Betäubung mit lsofluran/Luft erfolgte eine zusätzliche Lokalanästhesie durch i.c. Injektion von 2 % Xylocain unmittelbar in die für die Excision vorgesehene Stelle sowie die Entfettung der Haut mit Trichlorethylen. Daraufhin wurde den Mäusen jeweils eine kreisrunde Hautwunde mit einem Durchmesser von 1,3 cm mit einer Schere herausgeschnitten. Die Wunden wurden mit einem transparenten Pflaster (Hydrofilm, F. Hartmann) und Verbandskleber verschlossen. Über dieses Pflaster wurde ein weiteres, größeres Pflaster mit einer der Wunde entsprechenden Öffnung geklebt, die die weitere Beobachtung der Wunde ermöglicht. Durch das transparente Pflaster hindurch erfolgte die Applikation von:

3 x 200 kUnits BPP-Cys-PEG pro Tier in Methylcellulose und 5 % Maus-Serum (als Carrier) an den Tagen 0, Tag 2 und Tag 5.

Kontrollmäuse erhielten nur die Carriermischung. Die Mäuse wurden über einen Zeitraum von 29-30 Tagen beobachtet. Im Abstand von normalerweise fünf Tagen wurden die Pflaster gewechselt.

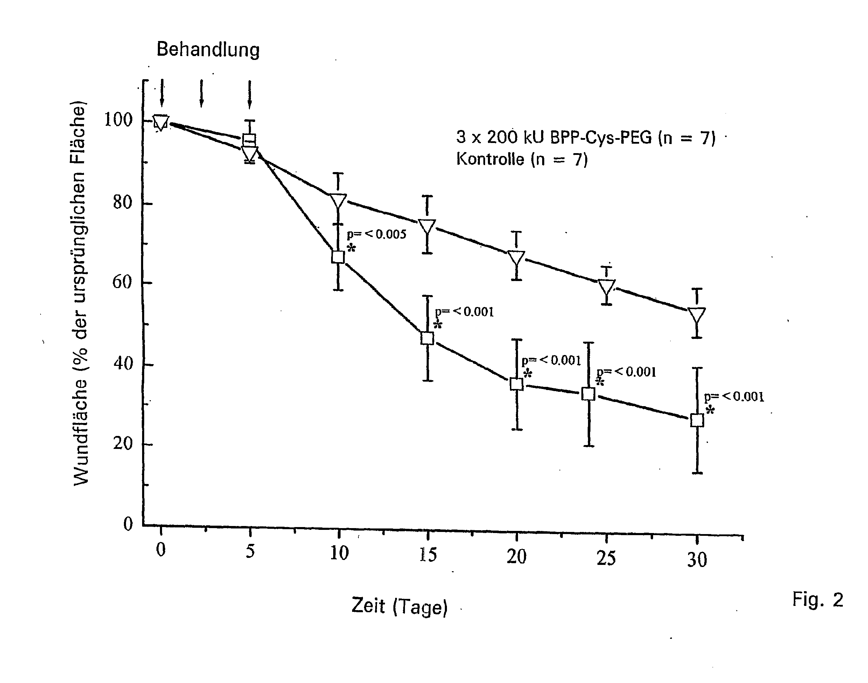

Durch die Applikation von BPP-Cys-PEG (3 x 200 kU) wurde eine signifikante Beschleunigung der Wundheilung erzielt (siehe Abbildung 2).

### 3. Verwendung von MALP-2-Derivaten bzw. BPP-Cys-PEG als mukosales Adjuvans für ein Hervorrufen einer wirksamen humoralen Antwort auf sowohl systemischem als auch mukosalem Niveau. Versuchsdurchführung:

6-8 Wochen alte weibliche BALB/c (H-2d) Mäuse (Harlan Winkelmann GmbH, Borchen, Deutschland) wurden für die Versuche verwendet. Gruppen von jeweils 5 Mäusen wurden am Tag 1, 14 und 21 mit 50 µg β-Galactosidase (β-gal) (Boehringer, Mannheim, Deutschland) alleine oder mit 0,5 µg synthetischen R-MALP-2, S-MALP-2 bzw. BPP-Cys-PEG als Adjuvans über den nasalen Weg verabreicht (25µl). Serumproben wurden am Tag 31 entnommen und bei -20 °C bis zur Bestimmung der β-gal spezifischen Antikörper gelagert. Nunc-Immuno MaxiSorp Testplatten mit 96 Vertiefungen (Nunc, Roskilde, Dänemark) wurden mit 100 µl β-gal (Boehringer, Mannheim, Deutschland) mit 5 µg/ml in 0,05 mol Carbonatpuffer (pH 8,2) pro Vertiefung beschichtet. Serielle zweifach Verdünnungen der Seren oder Spülungen in PBS mit 1 % BSA. und 0,05 % Tween 20 wurden hinzugefügt (100 µl/Vertiefung), und die Platten wurden zwei Stunden lang bei 37 °C inkubiert. Nach dem Waschen wurde biotinyliertes γ-kettenspezifisches Ziegen-Anti-Maus-lgG (Sigma Chemie, Deisenhofen, Deutschland) hinzugefügt, und die Platten wurden eine weitere Stunde lang bei 37 °C inkubiert. Nach viermaligem Waschen wurde 100 µl Peroxidasekonjugiertes Streptavidin (Pharmingen) zu den Vertiefungen hinzugegeben, und die Platten wurden 30 Minuten lang bei 37 C° inkubiert. Nach viermaligem Waschen wurden die Reaktionen mittels ABTS in 0,1 mol Citrat-Phosphatpuffer (pH 4,35), der 0,01 % H₂O₂ enthielt, entwickelt. Die Titer am Endpunkt wurden als der reziproke Log₂ der letzten Verdünnung, die nach 30-minütiger Inkubation eine optimale Dichte bei 405 nm von 0,1 Einheiten oberhalb der Werte der negativen Kontrollen nach 30-minütiger Inkubation ergab, dargestellt.

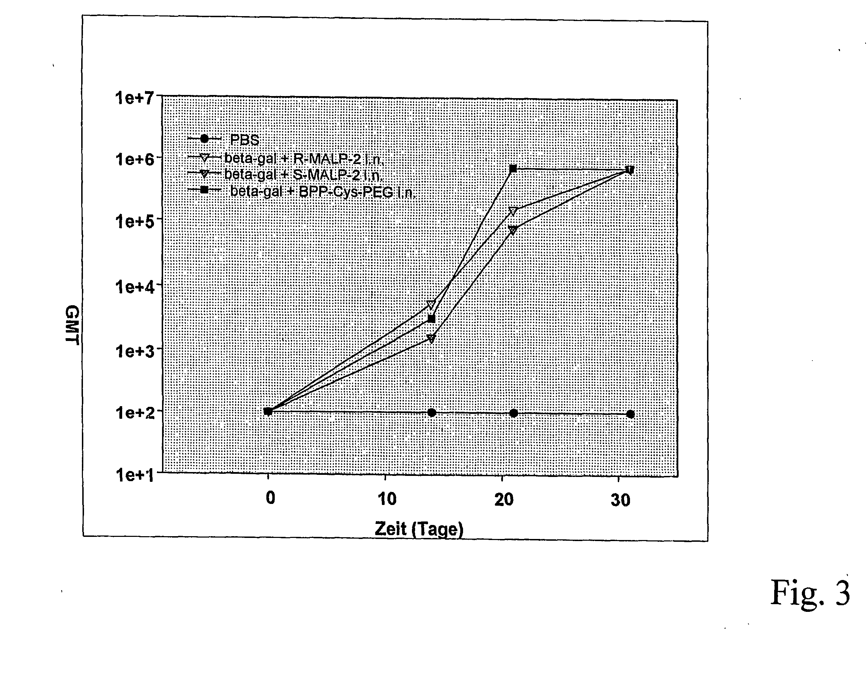

Es wurden Vaginal- und Lungenspülungen durch Spülen der Organe mit 1 mm PBS, das 50 mM EDTA, 0,1 % BSA und 10 mM PMSF enthielt, gewonnen. Die Spülungen wurden anschließend zentrifugiert, um Gewebetrümmer zu entfernen (10 min bei 3000 x g), und die Überstände wurden bei -20 °C aufbewahrt. Um die Konzentrationen an Gesamt-IgA der Spülungen der Lunge und Vagina zu bestimmen, wurden serielle Verdünnungen der entsprechenden Proben in Mikrotiterplatten inkubiert, wobei diese Platten zuvor mit Ziegen-Anti-Maus-lgA (Sigma Chemie, Deisenhofen, Deutschland) als Einfangantikörper (100 µl/Vertiefung) beschichtet wurden. Es wurden serielle Verdünnungen von gereinigtem Maus-1gA (Sigma Chemie, Deisenhofen, Deutschland) zum Erhalt einer Standardkurve verwendet. Um die Kapazität von R-MALP-2, S-MALP-2 und BPP-Cys-PEG zur Stimulierung von wirksamen humoralen Immunantworten zu untersuchen, wurden die Serumtiter von β-gal-spezifischen Antikörpern in den geimpften Mäusen bestimmt. Wie in Figur 3 dargestellt, führt die Verabreichung von β-gal in Anwesenheit von BPP-Cys-PEG zu einem Hervorrufen von antigenspezifischen IgG Titern, die schneller anstiegen als die mit Hilfe von R-MALP-2 erhaltenen.

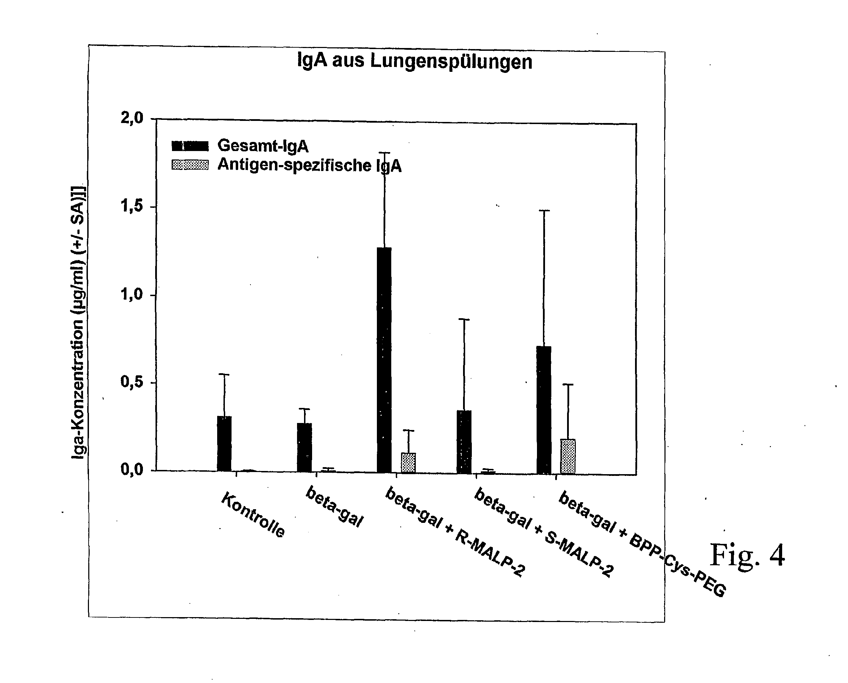

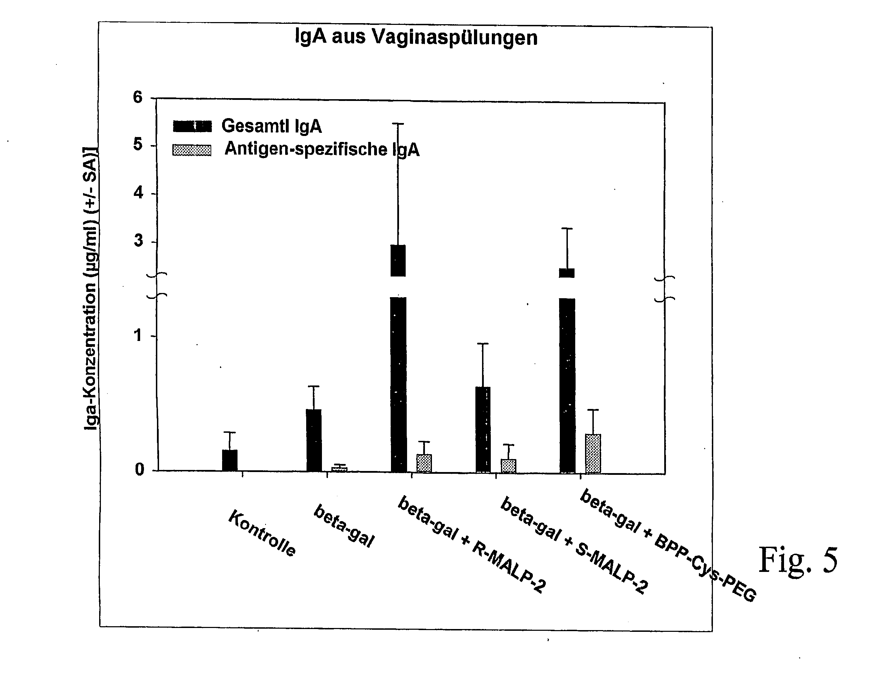

Um die Fähigkeit von obigen Derivaten zur Stimulation von Mukosalantworten gegenüber Antigenen, die intranasal (i.n.) zusammen wurden, zu bestimmen, wurde die Produktion von β-gal spezifischen IgA in Lungen- und Vaginalspülungen immunisierter Tiere untersucht. Während nach der i.n. Impfung mit reiner β-gal oder Mischungen mit dem S-MALP-2-Derivat keine Produktion von β-gal spezifischen IgA in den Lungenspülungen erfolgte, wurden bei den Tieren, die mit β-gal und zusätzlich entweder R-MALP-2 oder BPP-Cys-PEG immunisiert wurden, ein signifikanter Anstieg der antigenspezifischen IgA-Antikörper festgestellt (Figur 4). Die simultane Verabreichung von R-MALP-2 und BPP-Cys-PEG mit Antigen führte zur Stimulierung einer wirksamen IgA-Produktion auch an entfernt liegenden Schleimhäuten, wie dies durch die Anwesenheit signifikanter Konzentrationen von β-gal spezifischen IgA in vaginaler Spülungen nachgewiesen wurde (Figur 5).

### 4. Verwendung von BPP-Cys-PEG als mukosales Adjuvans führt zu einem Hervorrufen einer wirksamen T-Zell-vermittelten Proliferationsantwort

### Versuchsdurchführung:

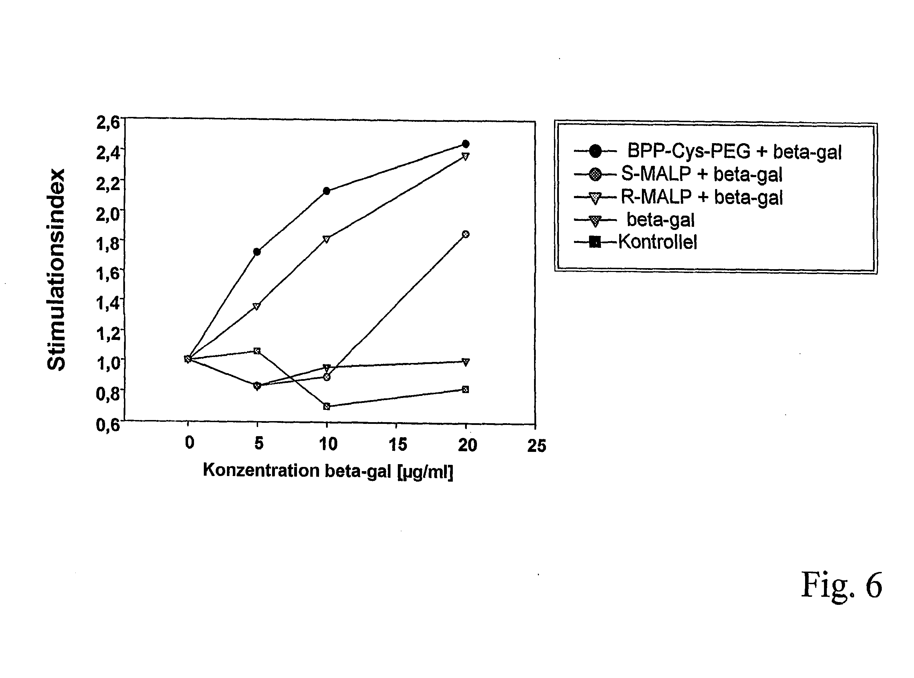

Die Milzen wurden entfernt und für die Analyse der Zellimmunantworten zusammengenommen. Die Zellen wurden in RPMI 1640, versetzt mit 10 % Kälberserum, 100 U/ml Penicillin, 15 µg/ml Streptomycin, 5 x 10⁻⁵ M 2-Mercaptoethanol und 1 mM L-Glutamin (GIBCO BRL, Karlsruhe, Deutschland) vermehrt und bei 37 °C in einer feuchten Atmosphäre mit 5 % CO₂ aufbewahrt. Die Zellsuspensionen wurden auf 5 x 10⁶ Zellen/ml im Komplettmedium eingestellt, in eine Flachbodenmikrotiterplatte mit 96 Vertiefungen (Nunc) mit 100 µl pro Vertiefung eingesät, und die Platten wurden 4 Tage lang in Anwesenheit von verschiedenen Konzentrationen löslichem β-gal inkubiert. Jede Konzentration wurde dreifach geprüft. Während der letzten 8-stündigen Kultivierung wurden zu jeder Vertiefung 1 µCi [³H]Thymidin (Amersham International, Freiburg, Deutschland) hinzugefügt. Diese Zellen wurden dann auf Papierfilter (Filtermat A; Wallac, Freiburg, Deutschland) mit Hilfe eines Zellernters (Inotech, Wohlen, Schweiz) geerntet und die Menge an in die DNA von poliferierenden Zellen aufgenommenem [³H]Thymidin wurde mittels Szintillationszähler (Wallac 1450, Micro-Trilux) bestimmt. Die Ergebnisse wurden als das Verhältnis zwischen den Werten (Durchschnittwert Dreifachbestimmung) von stimulierten und nicht-stimulierten Proben dargestellt (Stimulationsindex). Während die i.n. Verabreichung von β-gal alleine keine Induktion von nachweisbarer zellulärer Proliferation hervorrief, führte die simultane Verabreichung von R-MALP-2 oder BPP-Cys-PEG mit Antigen zu einer effektiven Proliferationsantwort (Figur 6). Es wird angemerkt, dass die stärkste T-Zell-Proliferationsantwort in Milzzellen von Mäusen immunisiert mit BPP-Cys-PEG und β-gal beobachtet werden konnte. Die Verwendung von S-MALP-2 führte zu einem wesentlichen schwächeren Stimulationsindex und nur bei Verwendung der höchsten Dosis von β-Galactosidase zur Restimulierung.

Die erhaltenen Ergebnisse zeigen deutlich, dass BPP-Cys-PEG als mukosales Adjuvans mindestens so wirksam oder sogar wirksamer ist als R-MALP-2. Wirksame humorale und zelluläre Immunantworten gegen simultan mit BPP-Cys-PEG verabreichte Antigene wurden sowohl auf systemischer als auch auf mukosaler Ebene erzielt.

## Patentansprüche

1. Bisacyloxypropylcystein-Konjugat gemäß Formel 1, 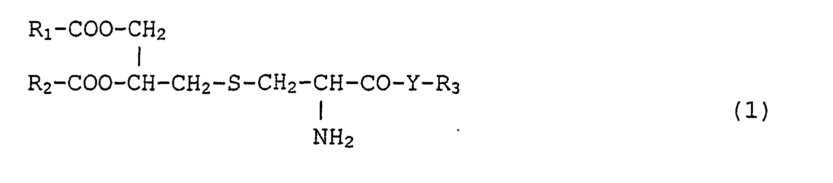 , wobei R₁ und R₂ gleich oder verschieden sein können und über die Carboxylgruppe gebundene Fettsäurereste bedeuten,
Y = -NH-, -O-, -S- oder -OCO- ist,
R₃ ein kovalent, ionisch oder assoziativ gebundener Konjugatrest ist, insbesondere ein wasserlösliches und physiologisch verträgliches, kovalent oder ionisch gebundenes Polymer, insbesondere kovalent gebundenes Polyethylenglykol (Polyoxyethylen),
-(CH₂-CH₂-O)ₘ-CH₂-CH₂-X ,
mit X = OR, NR₂, SR, COOR und
R = H, Benzyl-, C₁₋₆-Alkyl, wobei mehrere Reste R gleich oder verschieden sein können,
ein Polyoxyethylen-Polyoxypropylen-Copolymer, ein Dextran, ein Zucker, ein Polyvinylpyrrolidon, ein Alginat, ein Pektin oder ein Kollagen,
und wobei der polymere Rest R₃ ein-, zwei- oder mehrfach mit 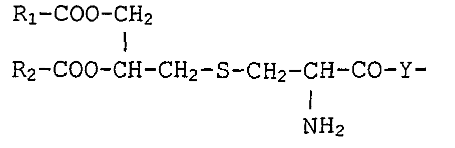 substituiert ist.

2. Bisacyloxypropylcystein-Konjugat nach Anspruch 1, **dadurch gekennzeichnet, dass** die Reste R_{1,2}, die gleich oder verschieden sein können, C₇₋₂₅-, vorzugsweise C₈₋₂₂-Alkyl, -Alkenyl oder -Alkinylgruppen sind und die ungesättigten Positionen vorzugsweise in cis-Konfiguration vorliegen, wobei die Alkyl-, Alkenyl -und Alkinyl-Reste verzweigte oder unverzweigte, cyclische oder cycloalkyl-substituierte Reste darstellen.

3. Bisacyloxypropylcystein-Konjugat nach Anspruch 1 oder 2, **dadurch gekennzeichnet, dass** das Molekulargewicht des wasserlöslichen Polymerrestes so gewählt ist, dass es pro Konjugat-Molekül 100 bis 30 000 Dalton beträgt.

4. Bisacyloxypropylcystein-Konjugat nach einem der Ansprüche 1 bis 3, **dadurch gekennzeichnet, dass** das Polyethylenglykol des Restes R₃ eine Kettenlänge m von 5 bis 700, vorzugsweise 100 bis 500 besitzt.

5. Bisacyloxypropylcystein-Konjugat nach einem der Ansprüche 1 bis 4, **dadurch gekennzeichnet, dass** die Verbindung ein S-[*2,3*-bis(acyloxy)-(2*S*)-propyl]-L-cysteinyl-carboxy-polyethylenglykol ist, vorzugsweise S-[*2,3-*bis(palmitoyloxy)-(2*S*)-propyl]-L-cysteinyl-carboxy-polyethylenglykol.

6. Bisacyloxypropylcystein-Konjugat nach einem der Ansprüche 1 bis 4, **dadurch gekennzeichnet, dass** die Verbindung ein S-[*2,3*-bis(acyloxy)-(2*R*)-propyl]-L-cysteinyl-carboxy-polyethylenglykol ist, vorzugsweise S-[*2,3-*bis(palmitoyloxy)-(2*R*)-propyl]-L-cysteinyl-carboxy-polyethylenglykol.

7. Pharmazeutische Zusammensetzung, enthaltend ein Bisacyloxypropylcystein-Konjugat nach einem der Ansprüche 1 bis 6.

8. Pharmazeutische Zusammensetzung nach Anspruch 7, **dadurch gekennzeichnet, dass** sie pharmazeutische Zusatz- oder Hilfsstoffe und vorzugsweise einen pharmazeutisch verträglichen Träger enthält.

9. Pharmazeutische Zusammensetzung nach Anspruch 7 oder 8 in Form einer für die Injektion, für die Inhalation oder für die intranasale oder topische Applikation geeigneten Formulierung.

10. Verwendung der Bisacyloxypropylcystein-Konjugate nach einem der Ansprüche 1 bis 6 oder der pharmazeutischen Zusammensetzung nach Anspruch 7, 8 oder 9 zur Stimulierung von Makrophagen, zur Stimulierung der Antikörper-Synthese, zur Infektionsabwehr, zur Immunostimulanz, insbesondere gegenüber Tumoren, zur Vorbeugung und Behandlung von septischem Schock, zur Wundheilung und als Adjuvans für Impfstoffe.
